# EUROPEAN PATENT SPECIFICATION

(11) **EP 3 787 734 B1**
(45) Date of publication and mention of the grant of the patent: **21.10.2026**
(21) Application number: 19796144.4
(22) Date of filing: 02.05.2019
(51) Int. Cl.: A61N 1/04, A61N 1/36

(54) **WEARABLE, ERGONOMIC NEUROSTIMULATION SYSTEM**
AM KÖRPER TRAGBARES, ERGONOMISCHES NEUROSTIMULATIONSSYSTEM
SYSTÈME DE NEUROSTIMULATION ERGONOMIQUE PORTABLE

(30) Priority: 03.05.2018 US 201862666647 P
(43) Date of publication of application: 10.03.2021
(60) Divisional application: 26201375.8
(73) Proprietor: Cala Health, Inc., San Mateo, CA 94404 (US)
(72) Inventor: LIBERATORE, Jessica, M., Burlingame, California 94010 (US); COLOMBO, John, Vincent, Burlingame, California 94010 (US); HAMNER, Samuel, Richard, Burlingame, California 94010 (US); ROSENBLUTH, Kathryn, H., Burlingame, California 94010 (US)
(74) Representative: J A Kemp LLP
(86) International application number: PCT/US2019/030458
(87) International publication number: WO 2019/213433

(56) References cited:
- WO-A1-2016/201366
- US-A1- 2003 093 098
- US-A1- 2013 158 627
- US-A1- 2013 158 627
- US-A1- 2013 296 967
- US-A1- 2017 157 398
- US-A1- 2017 157 398
- US-B2- 8 571 687

## Description

### BACKGROUND

### Field of the Invention

The invention relates generally to devices for electrically stimulating peripheral nerve(s) to treat various disorders. Without being claimed as such, the systems and methods as described herein can also include one, two, or more features as described, for example in U.S. Pat. 9,452,287 to Rosenbluth et al., U.S. Pat. No. 9,802,041 to Wong et al., PCT Pub. No. WO 2016/201366 to Wong et al., PCT Pub. No. WO 2017/132067 to Wong et al., PCT Pub. No. WO 2017/023864 to Hamner et al., PCT Pub. No. WO 2017/053847 to Hamner et al., PCT Pub. No. WO 2018/009680 to Wong et al., and PCT Pub. No. WO 2018/039458 to Rosenbluth et al..

### Description of the Related Art

Electrical energy can be delivered transcutaneously via electrodes on the skin surface with neurostimulation systems to stimulate peripheral nerves, such as the median, radial, and/or ulnar nerves in the upper extremities, or the tibial, saphenous, and/or peroneal nerve in the lower extremities as non-limiting examples. Electrical stimulation of these nerves has been shown to provide therapeutic benefit across a variety of diseases, including but not limited to movement disorders (including but not limited to essential tremor, Parkinson's tremor, orthostatic tremor, and multiple sclerosis), urological disorders, gastrointestinal disorders, cardiac diseases, and inflammatory diseases, among others. A number of conditions, such as tremors, can be treated through some form of transcutaneous peripheral nerve stimulation.

Other disorders can also be treated through peripheral nerve neurostimulation. For example, modulation of the sacral, saphenous, and/or tibial nerve could potentially improve symptoms of overactive bladder and urinary incontinence, and modulation of autonomic nerves, such as the vagus or tibial nerve relating to the parasympathetic nervous system, and/or any number of nerves associated with the sympathetic nervous system, could potentially improve symptoms of hypertension and cardiac dysrhythmias. Wearable systems with compact, ergonomic form factors are needed to enhance efficacy, compliance, and comfort with using the devices.

US 2013/158627A discloses an apparatus for transcutaneous electrical nerve stimulation in humans, comprising a housing; stimulation means mounted within the housing for electrically stimulating nerves; an electrode array releasably mounted to the housing, connectable to the stimulation means, and comprising electrodes for electrical stimulation of nerves; control means mounted to the housing and electrically connected to the stimulation means for controlling the stimulation means; monitoring means mounted to the housing and electrically connected to the stimulation means for monitoring the stimulation means; user interface means mounted to the housing and electrically connected to the control means for controlling the stimulation means; display means mounted to the housing and electrically connected to the control means and the monitoring means for displaying the status of the stimulations means; and a strap attached to the housing and configured to hold the housing, stimulation means and electrode array at a specific anatomical location to treat pain.

US 2017/157398A discloses a peripheral nerve stimulator can be used to stimulate a peripheral nerve to treat essential tremor, Parkinsonian tremor, and other forms of tremor. The stimulator can have electrodes that are placed circumferentially around the patient's wrist or arm. Specific nerves in the wrist or arm can be targeted by appropriate spacing of the electrodes. Positioning the electrodes on generally opposing sides of the target nerve can result in improved stimulation of the nerve. The stimulation pattern may alternate between the nerves. Improved stimulation algorithms can incorporate tremor feedback, external data, predictive adaptation, and long-term monitoring data.

WO 2016/201366A discloses a system and method for providing therapy to a patient which can include a monitoring unit and a therapy unit. The monitoring unit can have a user interface and one or more sensors to measure patient data. The therapy unit can have a stimulator for generating electrical stimulation and a microcontroller for controlling the generation of the electrical stimulation based on the measured patient data.

### SUMMARY

The presently-claimed invention is defined in claim 1. Further aspects and preferred embodiments are defined in the dependent claims. Any aspects, embodiments and examples of the present disclosure which do not fall under the scope of the appended claims do not form part of the presently-claimed invention and are merely provided for illustrative purposes.

In some embodiments, disclosed herein is a wearable neurostimulation device for transcutaneously stimulating one or more peripheral nerves of a user. The device can include any number of: an enclosure housing circuitry configured to generate electric stimulation signals, the enclosure having a top surface, a bottom surface, and a sidewall joining the top surface and the bottom surface; and an adjustable band configured to be worn by the user, the band having an inner side and an outer side, the inner side comprising at least one electrode for each nerve to be stimulated. In some embodiments, the enclosure can take an oval shape and includes curved top and bottom surfaces directly connected to each other, and does not comprise a separate sidewall therebetween. In some cases, an additional electrode can be present to serve as a return or counter electrode. In some cases, the enclosure can be removably attachable to the outer side of the band. The band can include an electrical interface member configured to both electrically and mechanically couple to the enclosure. In some embodiments, the electrical interface member allows for a direct connection between the enclosure and the band, and the electrical interface member does not comprise an external connection cable between the enclosure and the band. The bottom surface of the enclosure can include one or more electrical stimulation contacts. Each electrical stimulation contact can correspond to a different electrode of the band. The electrical stimulation contacts can be configured to be electrically connected to the electrical interface member. The enclosure can include one, two, or more stimulation buttons configured to start and stop delivery of the neurostimulation signals. In some embodiments, the stimulation button is positioned on the top surface of the enclosure. The stimulation button can be biased upward by a bracket spring feature. The bracket spring feature can include a generally flat surface and a plurality of spring fingers cut from the flat surface. The spring fingers can be configured to press upward on the stimulation button. The bracket spring can also be a cylindrical or annular spring element constructed from, for example, a compliant foam or polymer material. The enclosure can include one or more auxiliary interface features configured to allow the user to modulate one or more stimulation parameters. The one or more auxiliary interface features can be positioned on the sidewall of the enclosure. In some cases, the auxiliary interface features can be positioned on the top surface and/or bottom surface of the enclosure. The bottom surface of the enclosure can include one lifted edge on the longer side of the enclosure or two lifted edges positioned opposite one another. The lifted edge(s) can be configured to allow a user to insert his or her fingers between the bottom surface and the band in order for the user to grip the enclosure. The enclosure can include one or more charging ports configured to receive power from an external source to charge a rechargeable battery contained within the enclosure and an electrical connector configured to electrically connect the charging ports to the rechargeable battery. The same electrical connector can electrically connect the stimulation button to the circuitry configured to generate the stimulation signals. One or more charging ports can be positioned on a sidewall substantially opposite one or more auxiliary interface features. In some cases, one or more charging ports can be positioned on the bottom surface of the enclosure. The band can include at least a first electrode and a second electrode. The first electrode can be configured to stimulate the median nerve of the user and the second electrode being configured to stimulate the radial nerve of the user. The enclosure and the electrical interface member each can include ground or return electrode contacts configured to be electrically coupled to each other. The bottom surface of the enclosure can include a recess encompassing the one or more electrical stimulation contacts. The electrical interface member can be configured to be received within the recess to mechanically couple the enclosure to the band in a detachable manner. The electrical interface member and the enclosure can include corresponding keying features configured to ensure proper orientation of the enclosure when coupled to the band. for left handed or right handed wear. One advantage of such an embodiment is that a single hardware stimulation unit can be manufactured, and the screen flipped or otherwise adjusted in software to configure for right-handed or left-handed uses. The electrical stimulation contacts can project from the bottom surface of the enclosure and can be configured to be received within recesses of the electrical interface member via a snap fit. The enclosure can have a length, a width, and a height, and the length of the enclosure can be longer, shorter, or substantially the same as the width of the enclosure. The band can include a length and a width. The length of the band can be longer than the width of the band. The enclosure can be configured to be coupled to the band so that the length of the band is oriented substantially transverse to a length of the band. The length of the enclosure can be longer than the width of the band. The enclosure can be configured to be coupled to the band such that the length of the enclosure extends substantially further beyond a first side of the band than beyond a second side of the band opposite the first side. The band can also include an aperture on a first end of the band through which a second end of the band is inserted to form a closed loop. The second end of the band can include a fastener for securing the band to itself in an adjustable length manner. In some cases, the fastener is a hook and loop type fastener, magnetic fastener, or a clasp. In some cases, a locking flap can extend from the fastener to prevent the second end of the band from retreating through the aperture to open the closed loop. In some embodiments, the electrical stimulation contacts project from the bottom surface of the enclosure and are configured to be received within recesses of the electrical interface member via a rotatable connection. In some embodiments, the sidewall comprises a first surface and a second surface opposing the first surface, and the second surface is configured to be a bracing surface.

In some embodiments, disclosed herein is a wearable neurostimulation system. The neurostimulation system can include the neurostimulator and a charger configured to electrically charge the rechargeable battery of the enclosure. The charger can include a top surface, a bottom surface, and a sidewall extending between the top surface and the bottom surface. The charger can also include a charging pocket formed in the top surface. The pocket can be configured to receive at least a portion of the enclosure. The charging pocket can include one or more charging contacts at the bottom of the charging pocket configured to electrically couple to and transfer power to the enclosure, and a charging cable configured to be coupled to an external power source for drawing power through the charger. The charging pocket can also include an opening in the top surface forming a top face of the charging pocket. A cross-sectional area of the charging pocket can taper inward as the charging pocket extends downward from the opening, the taper being configured to help guide the enclosure into the charging pocket. The charging pocket can also be configured to receive the enclosure such that the longest dimension of the enclosure extends upward from the top surface of the charger and the smallest dimension of the enclosure faces outward from a center of the charger. The charging pocket can also be positioned off the center of the charger, and/or dimensioned so that the band rests upon the top surface of the charger when the enclosure is being charged. The system can also include one or more charger magnets, and the enclosure can include one or more corresponding magnets configured to be attracted to one or more charger magnets. The charger magnets and the corresponding magnets can be configured to properly align the enclosure with the charging contacts at the bottom of the charging pocket. The charger can also include two charging magnets positioned symmetrically around the charging contacts of the charging pocket, and the enclosure can include two corresponding magnets positioned symmetrically around one or more charging ports configured to receive charge from the charger. The bottom surface of the charger can include a cable pocket for storing the charging cable. The cable pocket can include a holding magnet for holding a free end of the charging cable within the cable pocket when in a stored position. The bottom surface can also include a rim extending around the perimeter of the cable pocket. The rim can include an opening forming an exit which allows the cable to extend from the cable pocket when charging such that the bottom surface of the charger may remain flat on a supporting surface. The band can include a RFID tag, and the charger can include an RFID antenna. The RFID tag can be configured to communicate an age of the band to the charger. The charger can be configured not to charge the enclosure if the age of the band. exceeds a threshold age, does not match a pre-determined identification, or other parameters. In some cases, the charger can include a first wireless communications antenna configured to receive and transmit data to and from a wireless communications antenna in the enclosure. In some cases, the RFID tag can be configured with a unique identifier associated with the patient or end user and the charger is configured to transmit or receive wireless data when the enclosure is in electrical connection with the charger and the appropriate RFID tag is in detected by an RFID antenna. In some cases, data transmitted between the enclosure and charger include but is not limited to device usage data, error data, motion or activity data, tremor motion data, and/or physiological data. In some cases, the charger can include a second wireless communications antenna configured to receive and transmit data from a system of remote servers, e.g., the cloud. In some cases, the charger is configured to transmit or receive wireless data only when the enclosure is in electrical connection with the charger and the appropriate RFID tag is detected by an RFID antenna. In some embodiments, the RFID tag is configured to communicate a unique personal identifier to the charger and the charger is configured not to charge the enclosure if the unique personal identifier is not recognized as valid by the charger. In some embodiments, the charger is configured to receive data from the enclosure when the enclosure is positioned in the charger, and wirelessly transmit the data to an external device.

Also disclosed herein is a wearable neurostimulation device for transcutaneously stimulating one or more peripheral nerves of a user. The device can include an enclosure housing circuitry configured to generate electric stimulation signals, the enclosure having a top surface and a bottom surface; and an adjustable band configured to be worn by the user, the band having an inner side and an outer side, the inner side comprising at least one electrode corresponding to each nerve to be stimulated. The band can comprise an electrical interface member configured to electrically and mechanically couple to the enclosure. The bottom surface of the enclosure comprises one or more electrical stimulation contacts, each electrical stimulation contact corresponding to a different electrode of the band, the electrical stimulation contacts configured to be electrically connected to the electrical interface.

In some embodiments, the enclosure is removably and directly attachable to the outer side of the band without a cable connector therebetween. In some embodiments, the electrical stimulation contacts project from the bottom surface of the enclosure and are configured to be received within recesses of the electrical interface member via a snap fit. In some embodiments, the electrical stimulation contacts project from the bottom surface of the enclosure and are configured to be received within recesses of the electrical interface member via a rotatable connection.

In some embodiments, the band comprises at least a first electrode and a second electrode, the first electrode being configured to stimulate the median nerve of the user and the second electrode being configured to stimulate the radial or ulnar nerve of the user. The enclosure and the electrical interface member can each comprise return electrode contacts configured to be electrically coupled to each other, and the bottom surface of the enclosure comprises a recess encompassing the one or more electrical stimulation contacts, wherein the electrical interface member is configured to be received within the recess to mechanically couple the enclosure to the band in a detachable manner.

In some embodiments, the band comprises an aperture on a first end of the band through which a second end of the band is inserted to form a closed loop, wherein the second end of the band comprises a hook and loop fastener for securing the band to itself in an adjustable length manner, and wherein a locking flap extends from the hook and loop fastener to prevent the second end of the band from retreating through the aperture to open the closed loop.

Also disclosed herein is a method of charging a wearable neurostimulation device for transcutaneously stimulating one or more peripheral nerves of a user. The method can include any number of providing the wearable stimulation device, comprising an enclosure and an adjustable band comprising an electrical interface member, the adjustable band configured to be electrically and mechanically coupled to the enclosure; detaching the adjustable band from the enclosure; connecting the enclosure to a charging station; verifying a unique identifier on the enclosure via the charging station; and wirelessly transmitting data received from the wearable stimulation device to an external device only while the enclosure is connected to the charging station. In some embodiments, the data is transmitted directly from the enclosure. In some embodiments, the data is transmitted from the enclosure to the charging station when the enclosure is connected to the charging station, and then wirelessly transmitted from the charging station to the external device. In some embodiments, wirelessly transmitting data does not occur when the enclosure is not connected to the charging station.

### BRIEF DESCRIPTION OF THE DRAWINGS

FIGS. 1A-1G schematically illustrate various views of an example of an enclosure configured for providing transcutaneous peripheral nerve stimulation.
FIGS. 2A-2B schematically illustrate various views of an example of a band configured to be worn by a user and to detachably couple to the enclosure for providing transcutaneous peripheral nerve stimulation to the user.
FIGS. 3A-3F schematically illustrate various views of an example of a charger configured to hold and receive a neurostimulation device for charging the neurostimulation device.
FIGS. 4A-4C are flow charts of methods for fitting and/or testing of a device, according to some embodiments of the invention.

### DETAILED DESCRIPTION

Disclosed herein are devices configured for providing neurostimulation. The neurostimulation devices provided herein are configured to stimulate peripheral nerves of a user. The neurostimulation devices are configured to transcutaneously transmit one or more neurostimulation signals across the skin of the user. The neurostimulation devices are wearable devices configured to be worn by a user, such as only unilaterally, or bilaterally utilizing a plurality of devices in some cases. The devices are configured to be worn on the wrist. In some embodiments, the neurostimulation devices do not include any implantable components (e.g., implantable under the skin, or elsewhere within a body). The user may be a human, another mammal, or other animal user.

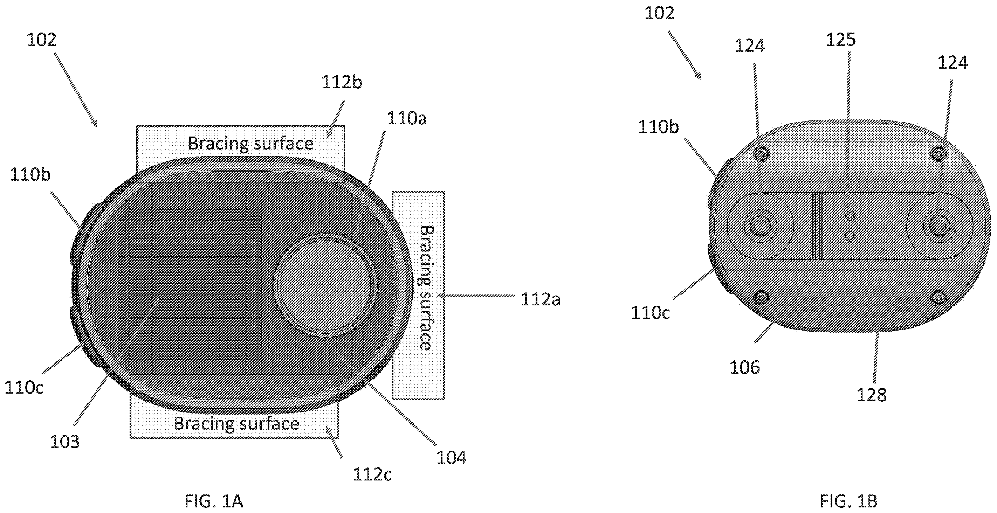

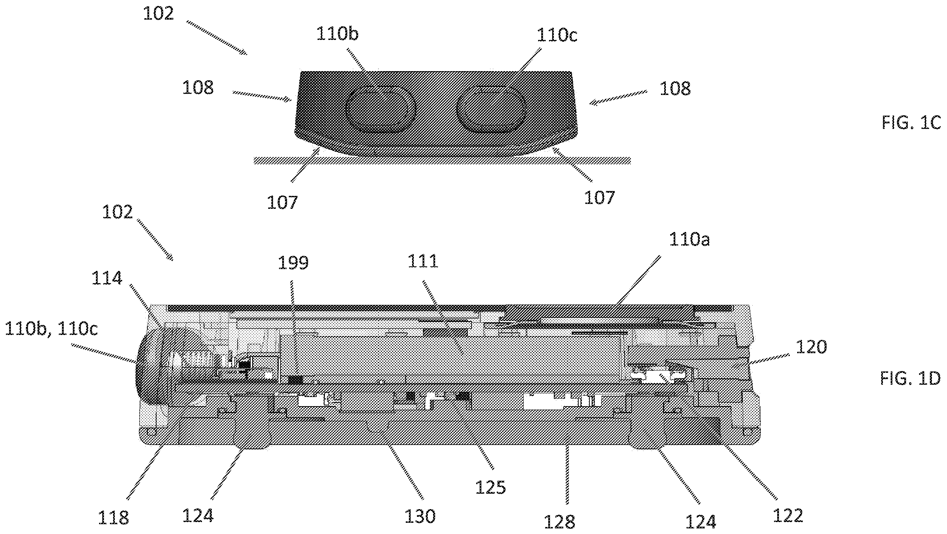

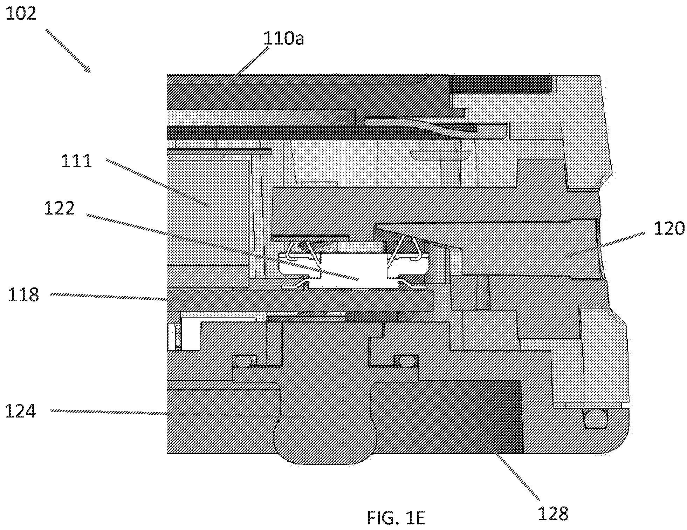

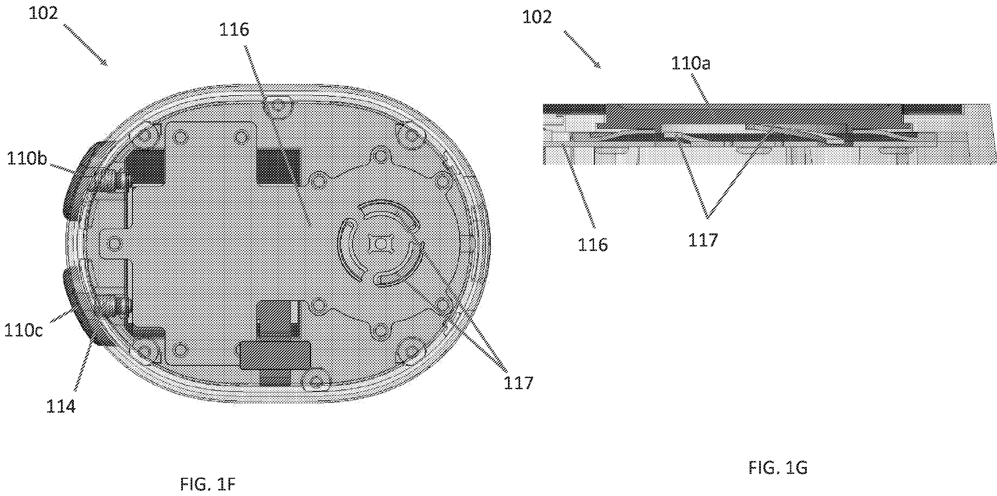

Figures 1A-1G depicts various views of an example of a body, housing, or enclosure 102 of a neurostimulation device 100. Figure 1A depicts a top view of the enclosure 102. Figure 1B depicts a bottom view of the enclosure 102 configured to face toward a skin surface of the user during neurostimulation. Figure 1C depicts a side view of the enclosure 102. Figure 1D depicts a cross-section of a side of the enclosure 102, which is substantially orthogonal to the side view depicted in Figure 1B. Figure 1E depicts a close-up view of the cross-section depicted in Figure 1D. Figure 1F depicts a top cross-section of the enclosure 102 including a spring feature. Figure 1G shows a close-up view of the cross-section depicted in Figure 1D including the spring feature shown in Figure 1G. The enclosure 102 has a top surface 104, as shown in Figure 1A, and an opposing bottom surface 106, as shown in Figure 1B. The enclosure 102 has a sidewall 108 extending from the top surface 104 to the bottom surface 106 and defining a height of the enclosure 102. The top surface 104 and the bottom surface 106 may have substantially the same shape. For example, the top surface 104 and bottom surface 106 may be substantially rectangular, substantially oval, or an intermediate shape between a rectangle and an oval. For example, the surfaces 104, 106 may comprise a substantially round, oval, or elliptical/ stadium-like shape, as shown in Figures 1A and 1B, or a rounded-corner rectangular or square shape. In other embodiments, the shape may be circular, triangular, polygonal, etc. The top surface 104 and the bottom surface 106 may have a length and a width, substantially transverse to the length. The length may be the same as the width or the length may be longer than the width. The top surface 104 and the bottom surface 106 may have substantially the same size. In some embodiments, the top surface 104 is slightly smaller than the bottom surface 106. For example, as shown in Figure 1C, the profile of the top surface 104 may be slightly reduced in size relative to the bottom surface 106 and positioned over the bottom surface 106 such that the sidewall 108 or a portion of the sidewall 108 forms an angled or contoured surface extending between the top surface 104 and the bottom surface 106. In some embodiments, the top surface has a dimension (e.g., length, width, and/or thickness) is larger or smaller by about or less than about 20%, 15%, 10%, 9%, 8%, 7%, 6%, 5%, 4%, 3%, 2%, 1%, or less of a dimension (e.g., length or width) of the bottom surface, or ranges including any two of the foregoing values.

The sidewall 108 may extend slightly inward as it extends upward from the bottom surface 106 to the top surface, by a distance of about or less than about 20%, 15%, 10%, 9%, 8%, 7%, 6%, 5%, 4%, 3%, 2%, 1%, or less of a dimension (e.g., length or width) of the sidewall 108, or ranges including any two of the foregoing values. The top surface 104 may be positioned centrally over top of the bottom surface 106 such that the entire sidewall 108 is angled or contoured along all edges of the enclosure 102. The contoured sidewall 108 may cause the size of the enclosure to appear smaller than it actually is.

The top surface 104 may be flat or substantially flat. In some embodiments, the top surface 104 may be somewhat convex or concave. The bottom surface 106 may be substantially flat. In some embodiments, the bottom surface 106 may be somewhat convex or concave. For example, in some embodiments, as shown in Figure 1C, the bottom surface 106 may have elevated or lifted edges 107 that are configured to extend above a central portion of the bottom surface 10 when the bottom surface 106 is resting on a flat surface. The lifted edges 107 may extend continuously (e.g., gradually) from a flat central portion of the bottom surface 106 to the edges of the bottom surface 106. In embodiments where the bottom surface comprises generally four edges, the bottom surface 106 may comprise 0, 1, 2, 3, or 4 lifted edges 107. In embodiments comprising two lifted edges 107, such as the embodiment shown in Figures 1A-1G, the two lifted edges 107 may be positioned substantially opposite each other. The lifted edges 107 may create lateral gaps between the bottom surface 106 and the surface the enclosure is resting on, such as a band and/or a body surface or body part of the user. The lateral gaps may create a grip space (e.g., finger space) and a portion of the bottom surface 106 by which a user can grip the enclosure 102. The grip space may facilitate the user detaching/removing the enclosure 102 from a band the enclosure is coupled to for allowing the user to wear the neurostimulation device 100. The lifted edges 107 may advantageously make the enclosure 102 appear visually smaller than it actually is. In other embodiments, the bottom surface 106 may be somewhat concave with edges that extend downward, such as to conform to the shape of a body part (e.g., a wrist) and aid in correctly positioning the device when worn. In some embodiments, the enclosure can take an oval shape and includes curved top and bottom surfaces, but does not include a separate sidewall.

The enclosure 102 may comprise a top member comprising the top surface 104 (or a majority thereof) and a bottom member comprising the bottom surface 106 (or a majority thereof). In some embodiments, the sidewall 108 may be formed as part of the top member and/or the bottom member. In some embodiments, the sidewall 108 may be formed from a separate shell component than the top member and the bottom member.

The enclosure 102 is configured to enclose or contain the electronic circuitry for generating and providing a neurostimulation signal to be applied to the user. The circuitry may be self-contained in the enclosure 102 such that the neurostimulation device 100 is portable. The circuitry may include a pulse generator for generating an electrical stimulation pulse and a controller for controlling the delivery of the electrical pulses. The enclosure 102 may also comprise a power source, such as a battery. The battery may be rechargeable and/or replaceable. In some embodiments, the battery may be a standard form battery. In some embodiments, the battery may be a proprietary battery. The enclosure 102 may also contain one or more processors. The enclosure 102 may also contain memory. The enclosure 102 comprises one or more displays (e.g., digital displays, LEDs, etc.) to display information to the user, such as on a top surface of the enclosure 102. Displays may also be touch-sensitive to receive inputs from the user. The enclosure 102 may comprise one or more audio signal generators. The enclosure may comprise antennas for wireless communication, such as Bluetooth, WiFi or Zigbee. The enclosure may also comprise a haptic motor to provide feedback or notification to the wearer by vibration. The enclosure 102 may comprise one or more interface features 110, such as depressable or solid state buttons for example, by which a user may interface with the neurostimulation device 100. For example, the user may use the interface features 110, to input parameters into the neurostimulation device 100, select a neurostimulation program stored on the neurostimulation device 100, to power off/on the neurostimulation device 100, and/or to begin, stop, or pause a neurostimulation treatment. The neurostimulation device 100 may comprise one, two, three, four, five, or more than five interface features.

As shown in Figure 1A, the enclosure 102 of the neurostimulation device 100 may comprise interface features or controls 110a, 110b, and 110c, which may be buttons, or touch-sensitive controls, dials, switches, or the like. The neurostimulation device 100 may comprise a first interface feature 110a on the top surface of the enclosure 102. The first interface feature 110a may be configured to start and stop neurostimulation. For instance, when pressed a first time, the first interface feature 110a may cause the neurostimulation device 100 to begin delivering a stimulation signal. When pressed a second time and held, the first interface feature 110a may cause the neurostimulation device 100 to cease delivering the neurostimulation signal. In some embodiments, the device could provide feedback such as visual, audible, or tactile (e.g., haptic feedback) upon activating a control, initiating or completing therapy, as an alarm when certain pre-determined criteria have been met, a vibration after a specific task is completed with the device, and the like. In some embodiments, the neurostimulation device 100 may deliver the neurostimulation signal indefinitely after the first interface feature 110a is activated. In some embodiments, the neurostimulation device 1 00 may be programmed to begin delivering a neurostimulation program (e.g., of a set duration of time or set number of pulses) after the first interface feature 110a is activated. In some embodiments, the duration of time could be about, at least about, or no more than about 5, 10, 15, 30, 40, 45, 60, 90, or 120 minutes, or ranges including any two of the foregoing values. In such embodiments, actuating (e.g., pressing) the first interface feature 110a again during the neurostimulation program may either pause or cancel the program. Actuating the first interface feature 110a a third time may resume or begin the neurostimulation program anew. Actuating the first interface feature 110a subsequently to completing the neurostimulation program may initiate another treatment session of the neurostimulation program. The first interface feature 110a may be a large button such that it is easy for the user to find and actuate. For instance, the first interface feature 110a may consume about, at least about, or no more than about 5%, 10%, 15%, 20%, 25%, 30%, 40%, 50%, or ranges including any two of the aforementioned values, or less than 5% or more than 50% the surface area of the top surface of the enclosure. The first interface feature 110a may be circular in shape, as shown in Figure 1A, or any other suitable shape (e.g., oval, rectangular, square, etc.).

As shown in Figures 1A-1C, the enclosure 102 of the neurostimulation device 100 may comprise one, two, or more auxiliary interface features 110b, 110c. The auxiliary interface features 110b, 110c may be the same or different type of interface feature 110 as the first interface feature 110a. For example, the auxiliary interface features 110b, 110c may be buttons. The auxiliary interface features 110b, 110c may be positioned on a sidewall of the enclosure 102 or on the top surface of the enclosure. The auxiliary interface features 110b, 110c may be positioned substantially adjacent to one another on a same side of the enclosure 102. The auxiliary features 110b, 110c may be configured to enter additional input, to modulate one or more stimulation parameters, sense a parameter relating to the patient, and/or to cause a display on the enclosure 102 to display selected information, and/or control another function. For example, the auxiliary interface features 110b, 110c may be used to increment and/or decrement a stimulation session duration, a stimulation voltage and/or current (intensity), and/or to select a stimulation program, or other customized functionality.

The size and/or shape of the enclosure 102 may be configured to provide one or more suitable bracing surfaces 112 for the user to actuate the interface features 110a, 110b, 110c, as shown in Figure 1A. For example, the various lateral surfaces of the sidewall 208 or lateral sides of the enclosure 102 may form bracing surfaces such as bracing surface 112a, 112b, 112c. The bracing surface 112a may be positioned substantially opposite the auxiliary interface features 110a, 110b. A user may brace his or her hand or one or more of his or her fingers against bracing surface 110a in order to advantageously provide a counter force that allows him or her to apply force in the direction of bracing surface 110a In order to actuate interface feature 110b and/or interface feature 110c. The bracing surfaces 110b and 110c may allow a user to apply slight compressive pressure to the enclosure 102 creating a frictional force which resists downward depression of the enclosure 102 and provides a counter force for the user actuating interface feature 110a by pressing it downward. The lifted edges 107 may be positioned under bracing surfaces 110a, 110b as shown in Figure 1C, which may allow a user to exert an upward force on the enclosure and similarly provide a counter force which allows actuation of interface feature 110a. The user may similarly use any suitable outer portion of the enclosure, including top surface 104, as a bracing feature 112 to actuate the one or more interface features 104. In some cases, a bracing surface can have a dimension, such as a length or are length of, for example, between about 20mm and about 80mm, between about 30mm and about 70mm, or between about 40mm and about 60mm. In some embodiments, a bracing surface can have a length or are length of about, at least about, or no more than about 20mm, 25mm, 30mm, 35mm, 40mm, 45mm, 50mm, 51mm, 52mm, 53mm, 54mm, 55mm, 56mm, 57mm, 58mm, 59mm, 60mm, 61mm, 62mm, 63mm, 64mm, 65mm, 70mm, 75mm, or 80mm, or ranges incorporating any two of the aforementioned values. In some embodiments, the length of a bracing surface can be about or at least about the length of a 95^{th} percentile male thumb (e.g., about 58mm), to provide stability for a majority of users of the device.

As shown in Figures 1D and 1F, the enclosure 102 may comprise springs 114 (e.g., coil springs) which bias the auxiliary interface features 114b, 114c laterally outward such that the buttons may be pressed inward against the force of the springs 114 and return to their pre-activated configuration when the pressure exerted by the user is released. In some embodiments, the first interface feature 114a may similarly be biased by a spring 114 biasing the first interface feature 114 upward. In some embodiments, the spring may be a cylindrical or annular foam or polymer element. In some embodiments, a different type of spring may be used. Figure 1F depicts an example of a spring feature 116 which may enable actuation of the first interface feature 110a. The spring feature 116 may comprise a thin bracket. The bracket spring feature 116 may be secured (e.g., bolted or screwed) to an internal component of the enclosure 102 around a periphery of the bracket. The bracket spring feature 116 may comprise a relatively expansive surface area to distribute the force from the actuation of the first interface feature 114a across a large area of the enclosure. The bracket spring feature 116 may be cut to form one or more finger springs 117 beneath the first interface feature 114a. The finger springs 117 may have elongate bodies that are cut or separated from the body of the bracket spring feature 116 along three sides, including two opposing elongate sides. The spring fingers 117 may be somewhat arcuate. A plurality of spring fingers 117 may be arranged substantially uniformly around a circumference of a circle to evenly support the weight of first interface feature 114a. The spring fingers 117 may be configured to extend upward away from the otherwise flat body of the bracket spring feature 116. Downward pressure exerted by the user on the first interface feature 114a may compress the spring fingers 110 downward until the spring fingers 117 are aligned flat with the body of the bracket spring feature 117, which prevents further downward movement of the first interface feature 114a. The spring fingers 117 may return the first interface feature 114a to an upward position after pressure exerted by the user is released from the first interface feature 114a. The use of a relatively flat bracket spring feature 116 may minimize the height of the enclosure 102 and allow the neurostimulation device 100 to conveniently maintain a low-profile with respect to the surface of the user's body. In other embodiments, one or more of the controls need not be physically movable and instead respond to touch feedback (e.g., capacitive or resistive feedback). In some embodiments, the controls can respond to voice commands, gestures using motion sensors or cameras, and the like.

Figure 1D schematically depicts at least some of the internal components housed within the enclosure 102. Figure 1E illustrates a close-up view of the right side of Figure 1D. The enclosure 102 may house a printed circuit board (PCB) 118 or equivalent circuitry. The enclosure 102 may include one or more charging ports 120 each configured to receive a charging pin, for example from a charger as described elsewhere herein, or an electrical stimulation contact from a charging cable. The enclosure 102 may comprise two charging ports 120 forming positive and negative terminals. Figure 1D and 1E illustrate an example of an enclosure comprising two charging ports 120 (one charging port 120 being hidden from view behind the other). In some embodiments, the enclosure may include a third charging port 120 (e.g., a ground port). In some embodiments, the enclosure can include one, two, or more ports to facilitate communications, such as a 1-wire interface for example. In some embodiments, the enclosure 102 may comprise an inductive coil for wirelessly powering a rechargeable battery 111. The charging ports 120 may be electrically connected to a rechargeable battery 111 via an electrical connector 122. The electrical connector 122 may be coupled to the PCB 118. In some embodiments, the same electrical connector 122 may electrically connect the first interface feature 114a to the PCB 118. The dual purpose of the electrical connector 122 may significantly simplify the manufacturing (e.g., the labor and cost) of the neurostimulation device 100. Some configurations as illustrated and described advantageously do not require the size of the enclosure to be increased.

As depicted in Figures 1B, 1D, and 1E, the enclosure 102 may include two or more electrical stimulation contacts 124 for delivering or transferring the electrical stimulation signal to the user or a downstream interface member. The electrical stimulation contacts 124 may be positioned on the bottom surface 106 of the enclosure 102. The electrical stimulation contacts 124 may include one electrical stimulation contact 124 for each electrode to be applied to the user. The electrical stimulation contacts 124 may include at least one electrical stimulation contact 124 for each nerve that is to be stimulated. For example, the electrical stimulation contacts 124 may include an electrical stimulation contact 124 configured to deliver a signal to the median nerve, the radial nerve, the ulnar nerve or any combination thereof. In some embodiments, stimulation may alternate between each nerve such that the nerves are not stimulated simultaneously. In some embodiments, all nerves are stimulated simultaneously. 1n some embodiments, stimulation is delivered to the various nerves in one of many bursting patterns. The stimulation parameters may include on/off, time duration, intensity, pulse rate, pulse width, waveform shape, and the ramp of pulse on and off. In one preferred embodiment the pulse rate may be from about 1 to about 5000 Hz, about 1Hz to about 500Hz, about 5 Hz to about 50Hz, about 50 Hz to about 300 Hz, or about 150 Hz. In some embodiments, the pulse rate may be from 1 kHz to 20 kHz. A preferred pulse width may range from, in some cases, 50 to 500 µs (micro-seconds), such as approximately 300 µs. The intensity of the electrical stimulation may vary from 0 mA to 500 mA, and a current may be approximately 1to 11 mA in some cases. The electrical stimulation can be adjusted in different patients and with different methods of electrical stimulation. The increment of intensity adjustment may be, for example, 0.1 mA to 1.0 mA. In one preferred embodiment the stimulation may last for approximately 10 minutes to 1hour, such as approximately 10, 20, 30, 40, 50, or 60 minutes, or ranges including any two of the foregoing values. In some embodiments, a plurality of electrical stimuli can be delivered offset in time from each other by a predetermined fraction of multiple of a period of a measured rhythmic biological signal such as hand tremor, such as about ¼, ½, or ¾ of the period of the measured signal for example. Further possible stimulation parameters are described, for example, in U.S. Pat. 9,452,287 to Rosenbluth et al., U.S. Pat. No. 9,802,041 to Wong et al., PCT Pub. No. WO 2016/201366 to Wong et al., PCT Pub. No. WO 2017/132067 to Wong et al., PCT Pub. No. WO 2017/023864 to Hamner et al., PCT Pub. No. WO 2017/053847 to Hamner et al., PCT Pub. No. WO 2018/009680 to Wong et al., and PCT Pub. No. WO 2018/039458 to Rosenbluth et al., each of the foregoing of which are hereby incorporated by reference in their entireties. In some embodiments, the device can also be configured to deliver magnetic, vibrational, mechanical, thermal, ultrasonic, or other forms of stimulation instead of, or in addition to electrical stimulation. However, in some embodiments, the device is configured to only deliver electrical stimulation, and is not configured to deliver one or more of magnetic, vibrational, mechanical, thermal, ultrasonic, or other forms of stimulation instead of, or in addition to electrical stimulation. The neurostimulation device 100, such as that depicted in Figures 1A-1G or others for example may include electrical stimulation contacts 124 configured to transcutaneously stimulate the median, ulnar, and/or radial nerves for example. As shown in Figure 1B, the enclosure may also include an optional return contact 125 for dispersing stimulation current from the body by returning to the stimulation source. Each contact 124, 125 may comprise one or more conductive pins or projections extending from the insulated enclosure 102. The contacts 124, 125 may be In electrical communication with the PCB 118. The bottom surface 106 may include a recess 128 configured to receive and mechanically couple to an interface member as described elsewhere herein. The recess 128 may encompass all the electrical contacts 124, 125. The recess may be round, oval, elliptical/stadium shaped, as shown in Figure 1B, or any other suitable shape. The electrical stimulation contacts 124 may be snap connections which form snap fits (e.g., annular snap fits) with corresponding contacts on an interface member comprising the electrodes to be applied to the user's skin. Other reversible connection mechanisms to connect the enclosure to the stimulation contacts can be utilized as well, including but not limited to magnets, screws, rotatable/rotational connection elements, an elastomer, and the like. The enclosure 102 and/or electrical connections may be configured such that the electrical stimulation contacts can be twisted into place within the enclosure 102. The electrical stimulation contacts 124 may be heat staked to the plastic enclosure 102, adhered to the enclosure 102 with a suitable adhesive, or otherwise firmly coupled or attached to the enclosure 102 by any suitable means known in the art. The electrical stimulation contacts 124 may be aligned with an axis extending the length of the enclosure 102. The axis may be centrally aligned along the enclosure 102. The ground contact 125 may be collinear with the electrical stimulation contacts 124. In some embodiments, the electrical stimulation contacts 124 may be spatially configured to stimulate the median, ulnar, and/or radial nerves, either in-line along the long axis of the patient's extremity, or circumferential around the band depending on the desired clinical result. In some embodiments, the electrodes corresponding to the electrical contacts 124 may be configured to be in electrical communication with the electrical contacts although arranged in a different spatial arrangement than the electrical contacts 124.

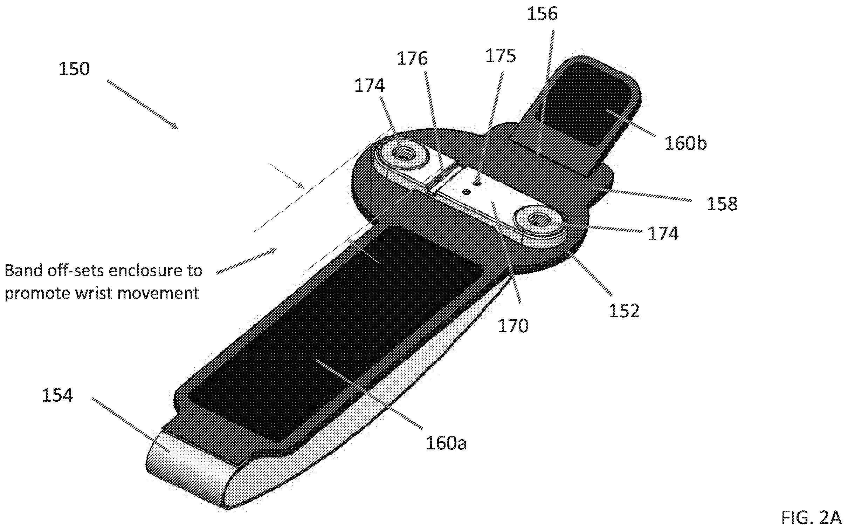

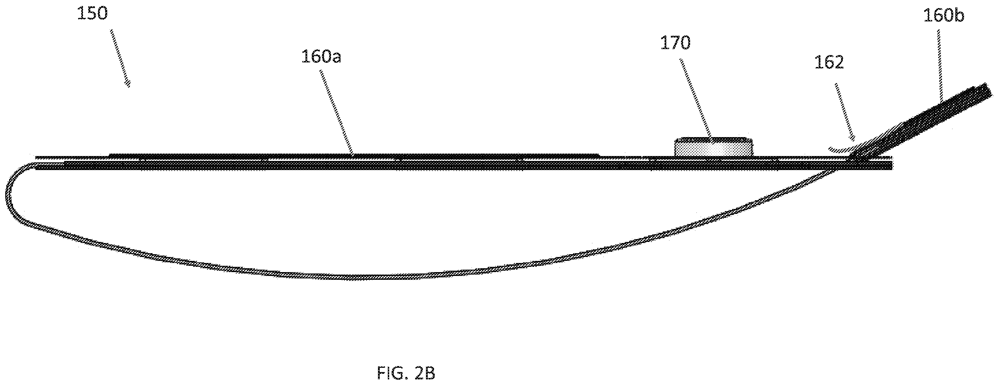

The enclosure 102 may be configured to be coupled to the surface of a user's skin for transcutaneous stimulation using a band 150. The electrodes could be percutaneous or microneedle electrodes in other embodiments, or only transcutaneous (e.g., not percutaneous, microneedles, or implanted electrodes in some embodiments). Figures 2A and 2B illustrate an example of a band 150 configured to be mechanically and electrically coupled to the enclosure 102 of Figures 1A-1G. The band 150 may be configured to be worn by a user around his or her arm, wrist finger, leg, ankle, knee, waist, etc. The band 150 may be configured to hold the enclosure 102 close to the user. In some embodiments, the band 150 may be configured as a D-shaped loop as shown in Figures 2A and 2B. The band 150 may comprise a base section 152 shaped and sized to sit underneath the enclosure 102. The band 150 may comprise a strap 154 extending from one side of the enclosure 102. In some embodiments, the strap 154 may have an adjustable length that is sufficient to accommodate any size user. In some embodiments, the strap 154 may be sized for various sizes of users (e.g., small, medium, large, child, adult, etc.). The strap 154 may have a width parallel to the length of the enclosure 102 and the base 12. The width of the strap 154 may be less than the length of the enclosure 102 and/or the corresponding length of the base 152. The length (the longer dimension) of the enclosure 102 may be oriented substantially perpendicular to the length of the strap 154 and may be configured to align the length of the enclosure 102 with the length of the user's arm, leg, or other body appendage. The alignment of the length of the enclosure 102 with the length of the body part may facilitate easier movement of the body part, such as the hand and wrist, while the neurostimulation device 100 is being worn and may be generally less protrusive and awkward and, therefore, less likely to snag or inadvertently contact something in the user's environment. In some embodiments, the strap 154 may be positioned substantially centrally along the length of the enclosure 102 and/or base 152. In some embodiments, as illustrated in Figure 2A, the strap 154 may be offset toward or near one side of the length of the enclosure 102 and/or base 152, preferably offset in a proximal direction relative to the wearer's limb. Offsetting the strap 154, may allow the strap 154 to be worn around, for example, the wrist of the user and the enclosure 102 to extend upward or proximally from the wrist in the direction of the shoulder rather than distally, or in the direction of the hand, which may beneficially allow or promote wrist movement (e.g., a larger range of motion).

The side of the base 152 of the band 150 opposite the strap 154 may comprise an aperture 156 (e.g., a D-loop) configured to receive the strap 154. The aperture 156 may be formed on a tab 158 extending from the base 152. The effective length of the strap 154 may be adjusted by pulling the strap further through the aperture 156. In some implementations the base 152, strap 154, and tab 158 of the band 150 may be fabricated as a single flat piece of flexible material. Fabricating these portions as a single piece of material may simplify the manufacturing process. Complementary sections of hook and loop fasteners 160 (e.g., Velcro^{™}) may be attached to the strap 154 for allowing the strap 154 to form a closed loop of an adjustable length for securing the band 150 to the user, for example around the user's arm, wrist, or leg. In some implementations, the band 150 may be fabricated by attaching the sections of hook and loop fasteners 160a, 160b to the strap after the strap has been received through the aperture 156. The strap 154 may comprise a large width portion and a small width portion. The small width portion may be configured to be received through the aperture 156. In some embodiments, one of the complementary sections of hook and loop fasteners 160a is attached to the large width portion (e.g., adjacent to the base 152) and the other section 160b is attached to the small width portion (e.g., at the free end of the strap 154). In some embodiments, the complementary hook and loop sections 160a, 160b may be affixed on the same side of the strap 154. For example, both sections 160a, 160b may be affixed to the outer surface of the strap 154, as shown in Figure 2A. The free end of the strap 154 may be wrapped over the enclosure 102 to join the complementary hook and loop sections 160a, 160b together. In some embodiments, the section 160a adjacent the base 152 may be affixed to the outer surface of the strap 154 and the section 160b at the free end of the strap 154 may be affixed to the inner surface of the strap 154, such that after looping through the aperture 166, the free end may double back on the strap to join the complementary hook and loop sections 160a, 160b together. The relative positioning of the complementary hook and loop sections 160a, 160b may be used to tighten or adjust the loop on the user's body.

In some embodiments, the hook and loop section 160b may be configured to prevent or inhibit the free end of the band 154 from retreating through the aperture 156. For instance, as schematically depicted in Figure 2B, the hook and loop section 160b may comprise a locking flap 162 extending from one side of the section configured to mechanically lock the free end on the outer surface of the band 150. An advantage of the locking flap on one side (e.g., only one side) is that the free end of the band can be easily inserted through the aperture, but cannot easily be removed or fall out when the user is donning the band. The flap requires a more intentional task of the user to remove, making the donning experience easier. The locking flap 162 may be a piece of the hook and loop section 160b material which is not adhered or affixed to the strap 154. The locking flap 162 may be an additional piece of material which is attached (e.g., sewn) onto the end of the hook and loop section 160b material. In some embodiments, the locking flap 162 may extend from a side of the hook and loop section 160b positioned opposite the free end of the strap 154, as shown in Figure 2B. In some embodiments, the locking flap 162 may extend in a direction opposite the free end of the strap 154, as shown in Figure 2B. In some embodiments, the locking flap 162 may extend from a side of the hook and loop section 160b positioned closest to the free end of the strap 154. In some embodiments, the locking flap 162 may extend in a direction toward the free end of the strap 154 (and possibly beyond the free end). In some embodiments, the hook and loop section 160b may comprise multiple locking flaps 162, such as a flap 162 extending from a side opposite the free end and extending away from the free end and a flap 162 extending from a side closest to the free end and extending toward the free end.

In some embodiments, the band 150 comprises an interface member 170 configured to mechanically and/or electronically interface with the electrical contacts 124, 125 protruding from the enclosure 102. Figure 2A and 2B depict an interface member 170 coupled to the base 152 of the band 150 and extending upward from an outer surface of the band 150. The interface member 170 may substantially mirror a portion of the bottom surface 106 comprising the electrical contacts 124, 125, as seen in Figure 1B. For instance, the interface member 170 may comprise electrical contact ports 174a, 174b configured to receive and electrically connect to the electrical stimulation contacts 124a, 124b of the enclosure 102 and electrical contact port 175 configured to receive and electrically connect to the ground contact 125 of the enclosure 102. In some embodiments, the electrical contacts may also provide a mechanical connection between the band and the enclosure. In some embodiments, electrical contacts are metallic, electrically conductive snap fasteners to provide a mechanical connection. The snap fasteners are comprised of a pair of interlocking discs made of metal where a circular tip under a first disc on the bottom surface of the enclosure that fits into a groove on the top surface of the second disc on the interface member of the band. The tip of the first disc is inserted into the groove of the second disc, holding the discs together until a certain amount of force is applied for removal. In some embodiments, the interface member does include a discrete cable connector. In alternative embodiments, the interface member 170 may comprise protruding electrical contacts and the enclosure 102 may comprise recessed electrical contacts or some of the electrical contacts on each the interface member 170 and the enclosure 102 may be protruding and some may be recessed. The device can also include one, two, three, or more sensors 199, which can include any number of combination of inertial measurement units (IMUs) single or multi-axis accelerometers, gyroscopes, inclinometers (to measure and correct for changes in the gravity field resulting from slow changes in the device's orientation), magnetometers; fiber optic electrogoniometers, optical tracking or electromagnetic tracking; electromyography (EMG) to detect firing of tremoring muscle; electroneurogram (ENG) signals; cortical recordings by techniques such as electroencephalography (EEG) or direct nerve recordings on an implant in close proximity to the nerve; heart rate or HRV sensors, galvanic skin response sensors, thermocouples, and/or other physiologic sensors, for example. While sensors 199 are shown schematically proximate the PCB 118 and battery 111, one or more sensors may be placed in other desired areas and proximate any of the illustrated and disclosed elements in some cases.

The interface member 170 may be configured (e.g., shaped and sized) to be received in the recess 128 of the enclosure 102. For example, the interface member may have any appropriate shape including those described elsewhere herein and a height matched to the depth of the recess 129. The interface member 170 may form a reversibly detachable interference fit or snap fit with the recess 128. In some embodiments, the interface member 170 may comprise a recess and the enclosure may comprise a projection. Positioning the protruding electrical contacts 124, 125 within the recess 128 may advantageously protect the electrical contacts 124, 125 from damage. In some embodiments, the enclosure 102 and the interface member 170 may comprise corresponding keying features which ensure the enclosure 102 and the interface member 170 are coupled in an appropriate orientation. For example, the enclosure 102 may comprise a ridge 130 extending from the bottom surface 106. The ridge 130 may be positioned between the electrical stimulation contacts 124. The ridge 130 may be positioned asymmetrically relative to the electrical stimulation contacts 124. The interface member 170 may comprise a channel 176 configured (e.g., sized and shaped) to receive the ridge 130. The keying features may ensure, for example, that the electrical stimulation contacts 124 are connected to the proper electrical contact ports 174 and not reversed. The keying features may be particularly advantageous for embodiments where the electrical contacts 124, 125 form a symmetric arrangement, as shown in Figure 1B. The keying features may ensure that the proper stimulation signal is electrically coupled to the proper electrode and, correspondingly, the proper nerve, and prevent the device from being worn on the wrong hand (e.g., right or left hand).

Any suitable mechanical coupling mechanism may be used to attach the enclosure to the band 150. The base 152 of the band 150 may comprise an aperture through which the interface member 170 extends upward from the outer surface of the band 150. The interface member 170 may be attached to the base 152 by any suitable means, such as an adhesive or permanent or removable mechanical fastener. The inner side of the interface member 170 may comprise electrodes or electrical contacts configured for transcutaneously stimulating the user. As described elsewhere herein, in some embodiments, there may be one electrode for each electrical stimulation contact 124. There may be one electrode for the ground contact 125. In some embodiments, the electrodes may be spatially arranged in the same manner as the electrical contacts 124, 125. In some embodiments, the electrodes may be arranged differently. For example, the electrodes may be arranged in a line substantially perpendicular to the line along which the electrical contacts 124, 125 are arranged, such that the electrodes are positioned, either axially and/or at least partially around a circumference of a body part (e.g., a wrist). In some embodiments, the electrodes may be configured to be generally in-line with the axon(s) of the target nerve being stimulated.

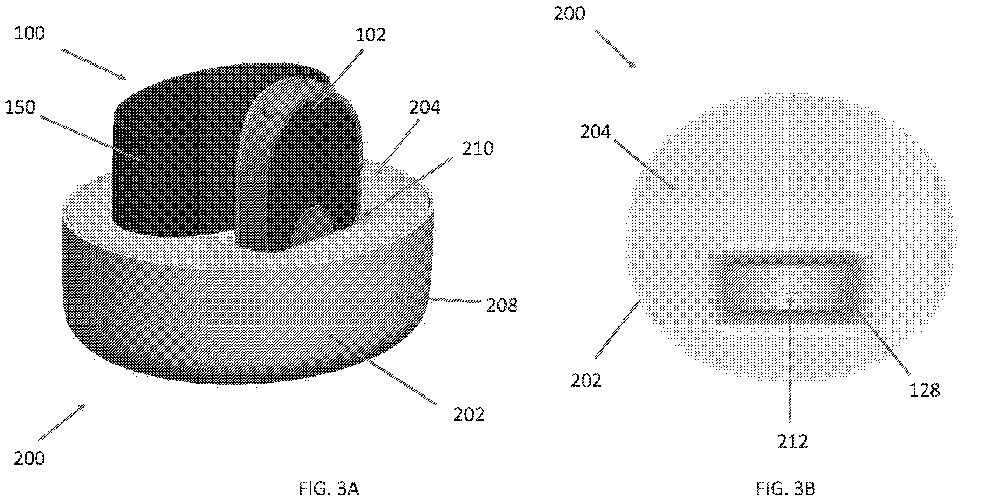

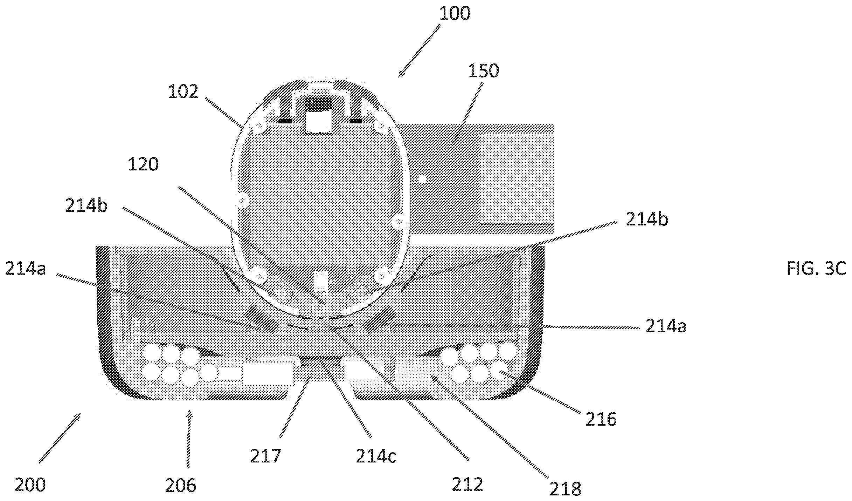

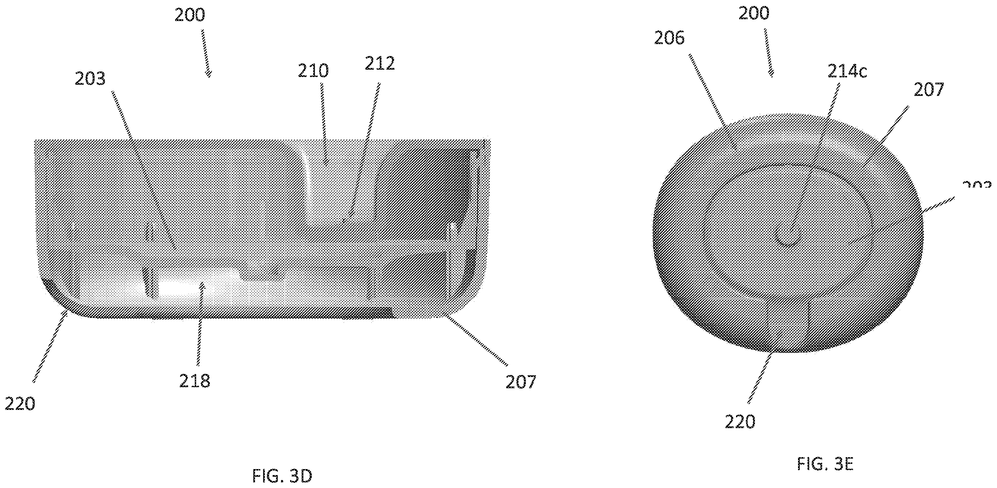

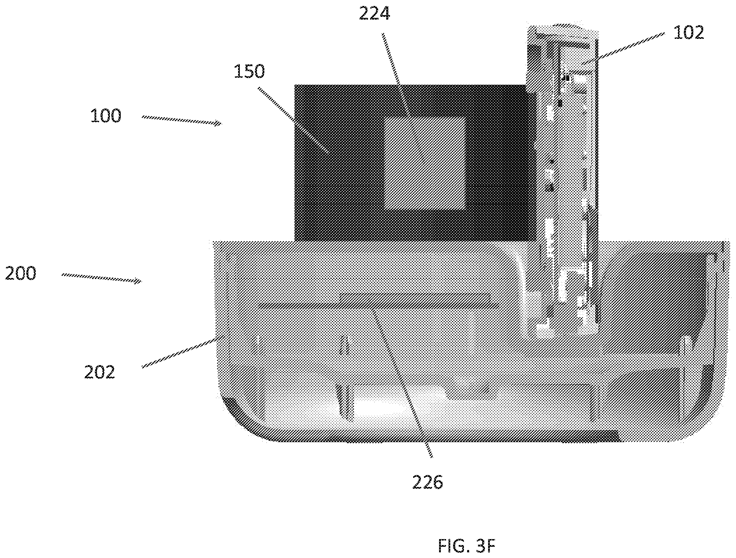

Disclosed herein is also a charger 200 configured for charging the neurostimulation device 100. Figures 3A-3F schematically depict an example of a charger 200. Figure 3A illustrates a perspective view of the charger 200 holding/charging the neurostimulation device 100. Figure 3B depicts a top view of the charger 200. Figure 3C depicts a cross-section of a side of the charger 200 holding/charging the neurostimulation device 100. Figure 3D depicts a cross-section of a side of the charger 200 which is substantially orthogonal to the side depicted in Figure 3C. Figure 3E illustrates a bottom view of the charger 200. Figure 3F depicts a cross-section of the same side of the charger 200 as depicted in Figure 3D but includes the neurostimulation device 100. The charger 200 may comprise a base receptacle 202 configured to receive and hold the neurostimulation device 100 while it recharges a rechargeable power source contained within the enclosure 102 of the neurostimulation device 100. The charger 200 may have a top surface 204, a bottom surface 206, and a sidewall 208. The top surface 204 and/or the bottom surface 206 may be substantially circular, round, or any other suitable shape. The charger 200 may generally be somewhat cylindrical. The charger 200 may comprise a rounded edge between the bottom surface 206 and the sidewall 208.

As seen in Figure 3B, the top surface 204 of the charger 200 may comprise a pocket 210 configured to receive and hold the device 100 for charging. The pocket 210 of the charger 200 may be configured to hold the neurostimulation device 100 in a substantially upright position in which length of the enclosure 102 is substantially normal to a top surface 204 of the charger 200. The charger 200 may be configured to orient the top surface 104 of the enclosure 102 radially outward from the center of the charger 200 and the bottom surface 106 radially inward toward the center of the charger. The depth of the pocket 210 may be any sufficient depth for stably holding the enclosure 102 in place. In some embodiments, the depth of the pocket 210 may be configured such that when the enclosure 102 is fully inserted into the pocket 210, the band 150 may rest on the top surface 204 of the charger 200 when the band 150 is attached to the enclosure 102. The configuration also may allow the band 150 to support the enclosure 102 location. In some embodiments, the band 150 may facilitate holding the enclosure 102 in an upright position. An advantage of keeping the enclosure in an upright position is that the enclosure pins maintain good contact with the charging pins in the charger. The enclosure 102 may also be charged while the band 150 is not attached to the enclosure 102. The top surface 204 may be substantially flat for supporting the band 150.
The pocket 210 may be positioned off the center of the top surface 204 of the charger 200 to leave space for the top surface 204 to accommodate the band 150. In some embodiments, the space for the band 150 may help the user properly orient the enclosure 102 when placing the enclosure 102 in the pocket 210. The pocket 210 may have a somewhat funneled or tapered opening such that the top face of the pocket 210 formed at the top of the pocket 210 on the top surface 204 is slightly larger than the bottom face of the pocket 210 formed at the bottom of the pocket 210. The sidewalls of the pocket may gradually taper inward from the top face of the pocket 210 to the bottom face of the pocket 210. In some embodiments, the taper is primarily or entirely concentrated at the top of the pocket 210. The taper may be advantageous for guiding the enclosure 102 into the pocket 210 such that the user does not need to as precisely target the pocket 210 as if the top face were sized to mirror the size of the enclosure 102. The bottom of the pocket 210 may more precisely match the size of the enclosure 102 to firmly hold the enclosure in place, which can advantageously provide a more reliable connection to the charging pins. In some embodiments, the band and/or enclosure does not include a discrete removable cable connector to connect to the charger.

As shown in Figure 3B, the bottom face of the pocket 210 may comprise one or more charging pins 212. The charging pins 212 may be configured (e.g., sized and shaped) and spaced to be received in the charging ports 120 of the enclosure 102. The charger 200 may be configured such that the charger automatically begins charging the enclosure 102 when the charging pins 212 are electrically coupled to the charging ports 120. In some embodiments, there may be two charging pins 212, such as a charging pin 212 forming a positive terminal and a charging pin 212 forming a negative terminal. In some embodiments, there may be an optional ground pin. In some embodiments, the enclosure can include one, two, or more ports to facilitate communications, such as a 1-wire interface for example as previously described. As shown in Figure 3C, the charger 200 may comprise one or more magnets 214a and the enclosure 102 may comprise one or more magnets 214b. The magnets 214a of the charger 200 may be paired with and complementary with (opposite polarity) the magnets 214b of the enclosure 102. In some embodiments, ferrous metal can be present in the enclosure 102 instead of magnets 214a to advantageously reduce weight in a wearable (e.g., wrist-worn device) and prevent attracting other ferrous metallic objects when worn. There may be 0, 1, 2, 3, 4, 5, or more than 5 pairs of magnets 214a, 214b. In some embodiments, as shown in Figure 3C, there may be two pairs of magnets 214a, 214b positioned across the width of the enclosure 102 and corresponding width of the pocket 210. The two pairs of magnets 214a, 214b may surround the charging pins 212. The pairs of magnets 214a, 214b may be arranged symmetrically across the enclosure 102 and the pocket 210. The magnets 214a may be positioned on or just underneath the surface of the pocket 210, as shown in Figure 3C. The magnets 214b may be positioned on or just underneath the surface of the enclosure 102, as shown in Figure 3C. The magnets 214a, 214b may ensure proper alignment of the charging ports 120 with the charging pins 212. The force between the pairs of magnets 214a, 214b, may be configured to stably hold or facilitate holding the enclosure 102 in place while allowing for release/removal of the enclosure by reasonable force applied from the user. In some embodiments, the magnets 214a, 214b may be strong enough to hold the enclosure 102 within the pocket 210 even when turned upside down. The use of magnets 214a, 214b may reduce the depth of the pocket 210 which is sufficient to firmly hold the enclosure 102 and may thereby reduce an overall height and size of the charger 200. In some embodiments, the charger includes user interface elements that indicate to the user that the enclosure is in good connection with the charger to charge the battery. In some embodiments, the user interface elements are LED lights. In some embodiments, the enclosure screen displays an icon and/or text to communicate that the device is properly charging.

As shown in Figure 3C, the charger 200 may comprise a charging cable 216 for drawing power from an external power source, such as, for example, an AC power outlet, or a computer or laptop device. The power cable 116 may be permanently attached to the base receptacle 202 at the point where it is electrically connected. In some embodiments, a strain relief feature is included on the permanently attached power cable at the point where it is electrically connected, having the advantage of preventing disconnection of the power cable when a force is applied to the cable. In some embodiments, the charger is connectable to the neurostimulation device (e.g., band and/or enclosure) via a connection element that does not comprise a cable. In some embodiments, the power cable 116 may be detachably connectable to the base receptacle 202 at the point where it electrically connects to the base receptacle 202. As shown in Figure 3C, the charging cable 216 may be a USB cable configured to receive power from a device comprising a USB port. The charger 200 may comprise a magnet 214c configured to magnetically hold in place a charging port 217 of the charging cable 216, such as in a compact position, when the charger 200 is not being used. Magnet 214c could be a discrete magnet from magnets 214a, 214b, or combined as part of the same magnet of 214a, 214b in some embodiments to advantageously reduce weight. The magnet 214c may have both positive and negative poles (not shown). The charging port 217 or a portion thereof may be metallic (e.g., a ferrous metal) such that it can be attracted to the magnet 214c. The charging port 217 may be easily released from the hold of the magnet 214c using nominal force by the user.

As shown in Figures 3C and 3D, the base receptacle 202 may comprise a hollow charging cable pocket 218 configured to store the charging cable 216 when it is not in use. The charging cable pocket 218 may comprise a bottom portion of the base receptacle 202. For example, the bottom surface 206 may be substantially opened, at least along a central portion of the bottom surface 206 for receiving the charging cable 216. The bottom surface 206 may comprise a rim 207 that surrounds the entire perimeter or at least a portion of the perimeter of the bottom surface 206 for facilitating retention of the charging cable 216 in a stored position, as seen In Figure 3E. The stored position may comprise the charging cable wrapped or coiled around a center of the base receptacle 202. In some embodiments, the height of the charging cable pocket 218 may be greater along an outside perimeter than along the center of the pocket 218 in order to promote storage of the charging cable 216 along the outside perimeter, as seen In Figure 3C. The charging cable pocket 218 may be configured (e.g., sized) such that the entire charging cable 216 may fit within the pocket 218 without extending across or below the bottom surface 206 such that the base receptacle 202 may sit flat on a surface when the charging cable 216 is in a stored position. In some embodiments, the rim 207 may comprise an exit 220, as seen in Figure 3E, or opening in the side of the rim 207. The exit 220 may be configured (e.g., sized) to allow a single width or diameter of the charging cable 216 to pass through the exit 220. The exit 220 may allow the charging cable 216 to be coupled to an external power source while the base receptacle 202 remains seated flat on a supporting surface. In some implementations, only the length of the charging cable 216 that is needed to reach the external power source may protrude from the exit 220. In some embodiments, the charging cable pocket 218 may be formed by inserting an insert 203 through the top of the base receptacle 202. The bottom of the insert 203 may form a ceiling of the charging cable pocket 218, as depicted in Figure 3F.

In some embodiments, the neurostimulation device 100 may comprise an RFID tag 224 or another suitable wireless communication mechanism known in the art. The charger 200 may comprise an RFID antenna 226, corresponding to the RFID tag 224, or another suitable wireless communication mechanism known in the art. The RFID tag 224 may be contained within (e.g., embedded within) the band 150, as shown in Figure 3F. The RFID antenna 226 may be contained internally within the base receptacle 202, as shown in Figure 3F. The RFID tag 224 may be configured to communicate information to the RFID antenna 226. In some embodiments, the RFID tag 224 may communicate an age or lifetime of the band 150 to the RFID antenna 226. In some embodiments, the RFID tag can be configured with a unique patient identifier that is associated with the base station, to ensure the device is being used by the person/patient it was intended or prescribed for. The age or lifetime of the band 150 may correspond to the age or lifetime of the stimulation electrodes coupled to the band 150. In some embodiments, the charger 200 may comprise a processor or circuitry coupled to the RFID antenna 226. The charger 200 may be configured to prevent charging of the enclosure 102 if the age or lifetime of the band 150 exceeds a threshold limit. In some embodiments, an RFID tag or another digital identification mechanism of a device can prevent operation/charging if it is not operably coupled to its uniquely-identified charger, to prevent swapping of devices or bands (which may be custom configured to treat that specific patient) between patients. The threshold limit (which can be, for example, no more than about 1 week, 2 weeks, 3 weeks, 4 weeks, 2 months, 3 months, 4 months, 5 months, or 6 months in some cases) may prevent using a band 150 beyond the usable lifetime of the band (e.g., the useable lifetime of the electrodes), which may degrade in performance over time and reduce the therapeutic efficacy of stimulation. In some embodiments, the charger 200 may include one or more indicators (e.g., LEDs). The LEDs (not shown) may provide visual indicator signals to indicate the status of the charging to the user. For instance, the LED may shine yellow when charging. The LED may shine green when the enclosure 102 is fully charged. The LED may shine red when the enclosure 102 is coupled to the charger 200, but the charger 200 is not charging the enclosure 102. Non-charging indicators may apply, for example, when the detected age of the band 150 exceeds the threshold limit and/or when another fault condition is detected (e.g., an improper external power source, such as one that exceeds voltage or current limits). In some embodiments, multiple LEDs may be used. In some embodiments, audible or tactile (e.g., haptic) indicators may be used.

In some cases, the charger includes a base station, and can include a first wireless communications antenna configured to receive and transmit data to and from a wireless communications antenna in the enclosure. In some cases, the RFID tag can be configured with a unique identifier associated with the patient or end user and the charger is configured to transmit or receive wireless data when the enclosure is in electrical connection with the charger and the appropriate RFID tag is in detected by an RFID antenna. In some cases, data transmitted between the enclosure and charger include but is not limited to device usage data, error data, motion or activity data, tremor motion data, and/or physiological data. In some cases, the charger can include a second wireless communications antenna configured to receive and transmit data from a system of remote servers, e.g., the cloud. In some cases, the charger is configured to transmit or receive wireless data only when the enclosure is in electrical connection with the charger and the appropriate RFID tag is detected by an RFID antenna. In some embodiments, the enclosure and/or the band is configured to transmit data to the base station of the charger while directly connected to the charger, and lacks any wireless communication capability. The base station of the charger includes the wireless communication capability to transmit the data to an external device. In some embodiments, such features can be advantageous to control bandwidth of data transmitted to an external device, e.g., the cloud and only done when device is not worn (e.g., connected to the base station of the charger). This can improve safety and security. Additionally, requiring multiple physical checks for data transmission can add layers of security to prevent hacking of data or device via the wireless communication connection.

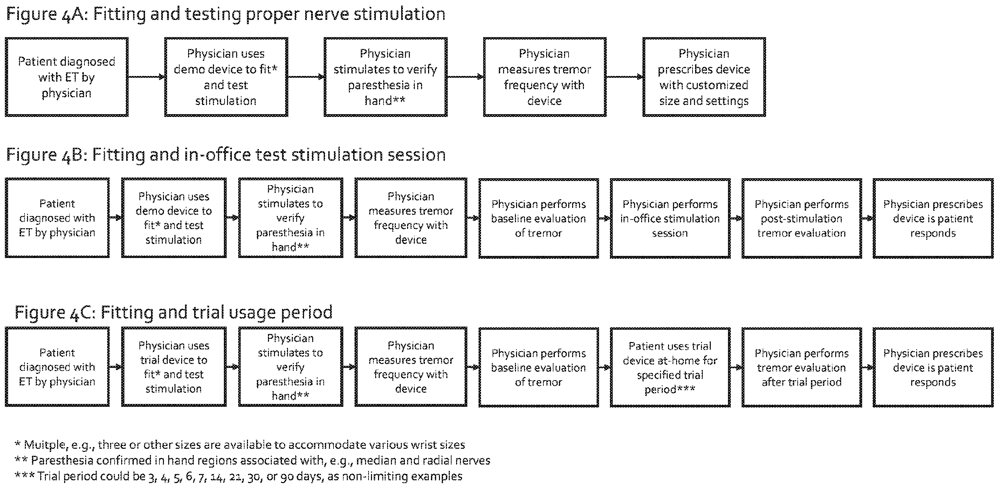

Figures 4A-4C are flow charts of methods for fitting and/or testing of a device, according to some embodiments of the invention. As shown in Figure 4A, a method for fitting and testing proper nerve stimulation can include any number of the following. A patient can be diagnosed with a medical condition, such as essential trenior for example. The physician can use a demo device to fit and test stimulation. The demo device can come in multiple sizes/configurations to accommodate various wrist sizes. The physician can activate the device stimulation features to stimulate one, two, or more nerves of the patient, and adjust stimulation intensity (e.g., current amplitude) to verify that paresthesia is present in the anatomical location(s) of interest (e.g., the hand, such as the areas innervated by the median and radial nerves, for example). Paresthesia can be verified, for example, by asking the patient if they feel numbness and tingling in the locations of interest after activation of the device. The physician can measure the tremor frequency, period, and/or amplitude with the device. The physician can then prescribe a device with customized size and settings.

As shown in Figure 4B, a method for fitting and in-office test stimulation can include any number of the following. A patient can be diagnosed with a medical condition, such as essential tremor for example. The physician can use a demo device to fit and test stimulation. The demo device can come in multiple sizes/configurations to accommodate various wrist sizes. The physician can activate the device stimulation features to stimulate one, two, or more nerves of the patient, and adjust stimulation intensity (e.g., current amplitude) to verify that paresthesia is present in the anatomical location(s) of interest (e.g., the hand, such as the areas innervated by the median and radial nerves, for example). The physician can measure the tremor motion to calculate tremor frequency, period, and/or amplitude with the device. The physician can then perform a baseline evaluation of tremor. The physician can perform an in-office stimulation session. The physician can then perform a post-stimulation tremor evaluation to determine the response to therapy. The physician can prescribe a device with customized size and settings depending on the degree of response to the in-office stimulation session and other factors.

As shown in Figure 4C, a method with a fitting and trial usage period can include any number of the following. A patient can be diagnosed with a medical condition, such as essential tremor for example. In some cases, the physician can use a trial device to fit and test stimulation. The trial device can have multiple sizes/configurations to accommodate various wrist sizes. The physician can activate the device stimulation features to stimulate one, two, or more nerves of the patient, and adjust stimulation intensity (i.e., current amplitude) to verify that paresthesia is present in the anatomical location(s) of interest (e.g., the hand, such as the areas innervated by the median and radial nerves, for example). The physician can measure the tremor frequency, period, and/or amplitude with the device. The physician can then perform a baseline evaluation of tremor. The patient can then use the trial device at home for a specified trial period, such as, for example, about 1, 2, 3, 4, 5, 6, 7, 14, 21, 30, 60, 90, or more days, or ranges including any two of the aforementioned values. The physician can then perform a post-stimulation tremor evaluation to determine the response to therapy. In some cases, the device can measure tremor motion post-stimulation to quantify tremor amplitude and transmit that information to the patient or physician. The physician can prescribe a device with customized size and settings depending on the degree of response to the at-home trial stimulation session and other factors.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +,'- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description. The methods disclosed herein include certain actions taken by a practitioner; however, they can also include any third-party instruction of those actions, either expressly or by implication. For example, actions such as "percutaneously stimulating an afferent peripheral nerve" includes "instructing the stimulation of an afferent peripheral nerve."

## Claims

1. A wearable neurostimulation device (100) for transcutaneously stimulating one or more peripheral nerves of a user, the device comprising:
an enclosure (102) housing circuitry (118) configured to generate electric stimulation signals, the enclosure having a top surface (104), and a bottom surface (106); and
an adjustable band (150) configured to be worn around a wrist by the user, the band having an inner side and an outer side, the inner side comprising at least one electrode for each nerve to be stimulated;
wherein the enclosure (102) is removably attachable to the band (150);
wherein the top surface (104) is configured to face away from the wrist of the user when the enclosure is attached to the band and the band is worn around the wrist of the user;
wherein the top surface (104) comprises a screen configured to display information to the user;
wherein the bottom surface (106) is configured to face towards the wrist of the user when the enclosure is attached to the band and the band is worn around the wrist of the user;
wherein the band (150) comprises an electrical interface member (170) and wherein the band is configured to be electrically and mechanically coupled to the enclosure (102);
wherein the enclosure (102) comprises one or more electrical stimulation contacts (124), each electrical stimulation contact corresponding to a different electrode of the band, the one or more electrical stimulation contacts configured to be electrically connected to the electrical interface member; and
wherein the enclosure comprises a stimulation button (110) configured to start and stop delivery of the stimulation signals;
**characterized in that**
the band comprises an RFID tag (224) configured to store information relating to an age or lifetime of the band so as to prevent charging of the enclosure if the age or lifetime of the band exceeds a threshold limit.

2. The neurostimulation device (100) of claim 1, wherein the stimulation button (110) is positioned on the top surface (104) of the enclosure (102).

3. The neurostimulation device (100) of claim 2, wherein the stimulation button (110) is biased upward by a spring feature (114, 116) and wherein the spring feature is a bracket spring feature, the bracket spring feature comprising a generally flat surface and a plurality of spring fingers (117) cut from the flat surface, the spring fingers configured to press upward on the stimulation button (110).

4. The neurostimulation device (100) of any of the preceding claims, wherein the enclosure (102) comprises one or more auxiliary interface features (110b, 110c) configured to allow the user to modulate one or more stimulation parameters, the one or more auxiliary interface features being positioned on the top surface (104) of the enclosure.

5. The neurostimulation device (100) of any one of the preceding claims, wherein the enclosure (102) comprises one or more charging ports (120) configured to receive power from an external source to charge a rechargeable battery (111) contained within the enclosure and further comprising an electrical connector (122) configured to electrically connect the one or more charging ports to the rechargeable battery, wherein the same electrical connector electrically connects the stimulation button (110) to the circuitry (118) configured to generate the stimulation signals.

6. The neurostimulation device of claim 1, wherein the enclosure comprises an inductive coil for wirelessly powering a rechargeable battery (111) contained within the enclosure.

7. The neurostimulation device (100) of any one of the preceding claims, wherein the enclosure (102) and the electrical interface member (122) each comprise return electrode contacts configured to be electrically coupled to each other.

8. The neurostimulation device (100) of any one of the preceding claims, wherein the electrical interface member (1220 and the enclosure comprise (1020 corresponding keying features configured to ensure proper orientation of the enclosure when coupled to the band.

9. The neurostimulation device (100) of any one of the preceding claims:
wherein the enclosure (102) has a length, a width, and a height, the length of the enclosure being longer than the width of the enclosure;
wherein the band (150) has a length and a width, the length of the band being longer than the width of the band; and
wherein the enclosure (102) is configured to be coupled to the band (150) so that the length of the band is oriented substantially transverse to a length of the band.

10. The neurostimulation device (100) of any one of the preceding claims, wherein the band (150) comprises an aperture (156) on a first end of the band through which a second end of the band is inserted to form a closed loop, wherein the second end of the band comprises a hook and loop fastener for securing the band to itself in an adjustable length manner, and wherein a locking flap extends from the hook and loop fastener to prevent the second end of the band from retreating through the aperture to open the closed loop.

11. The neurostimulation device (100) of any one of the preceding claims, further comprising a sidewall (108) joining the first surface and the second surface, wherein the sidewall (108) comprises a first sidewall surface and a second sidewall surface opposing the first sidewall surface, wherein the second sidewall surface is configured to be a bracing surface (112).

12. A wearable neurostimulation system, the neurostimulation system comprising the neurostimulation device (100) of claim 5 or any of claims 6-11 when dependent on claim 5, the system further comprising a charger (200) configured to electrically charge the rechargeable battery (111) of the enclosure, the charger comprising:
a first charger surface (204), a second charger surface (206), and a sidewall (208) extending between the first charger surface and the second charger surface;
a charging pocket (210) formed in the first charger surface, the pocket configured to receive at least a portion of the enclosure (102), the charging pocket comprising one or more charging contacts (212) at the bottom of the charging pocket configured to electrically couple to and transfer power to the enclosure;
and a charging cable configured to be coupled to an external power source for drawing power through the charger.

13. The neurostimulation system of claim 12, wherein the charging pocket (210) comprises an opening in the first charger surface (204) forming a top face of the charging pocket, and wherein a cross-sectional area of the charging pocket tapers inward as the charging pocket extends downward from the opening, the taper being configured to help guide the enclosure (102) into the charging pocket.

14. The neurostimulation system of claim 12 or 13, wherein the charging pocket (210) is configured to receive the enclosure (102) such that the longest dimension of the enclosure extends upward from the first charger surface (104) of the charger and the smallest dimension of the enclosure faces outward from a center of the charger.

15. The neurostimulation system of any one of claims 12 to 14, wherein the charging pocket (210) is positioned off the center of the charger and wherein the charging pocket is dimensioned so that the band rests upon the first charger surface (204) of the charger when the enclosure (102) is being charged.

## Patentansprüche

1. Tragbare Neurostimulationsvorrichtung (100) zur transkutanen Stimulation eines oder mehrerer peripherer Nerven eines Benutzers, wobei die Vorrichtung umfasst:
ein Gehäuse (102), in dem eine Schaltung (118) untergebracht ist, die dafür konfiguriert ist, elektrische Stimulationssignale zu erzeugen, wobei das Gehäuse eine Oberseite (104) und eine Unterseite (106) aufweist; sowie
ein verstellbares Band (150), das so konfiguriert ist, dass es vom Benutzer um das Handgelenk getragen werden kann, wobei das Band eine Innenseite und eine Außenseite aufweist, wobei die Innenseite mindestens eine Elektrode für jeden zu stimulierenden Nerv umfasst;
wobei das Gehäuse (102) abnehmbar an dem Band (150) befestigbar ist;
wobei die Oberseite (104) so konfiguriert ist, dass sie vom Handgelenk des Benutzers weg zeigt, wenn das Gehäuse am Band befestigt ist und das Band um das Handgelenk des Benutzers getragen wird;
wobei die Oberseite (104) einen Bildschirm umfasst, der so konfiguriert ist, dass er dem Benutzer Informationen anzeigt;
wobei die Unterseite (106) so konfiguriert ist, dass sie zum Handgelenk des Benutzers hin zeigt, wenn das Gehäuse am Band befestigt ist und das Band um das Handgelenk des Benutzers getragen wird;
wobei das Band (150) ein elektrisches Schnittstellenelement (170) umfasst und wobei das Band so konfiguriert ist, dass es elektrisch und mechanisch mit dem Gehäuse (102) gekoppelt ist;
wobei das Gehäuse (102) einen oder mehrere elektrische Stimulationskontakte (124) umfasst, wobei jeder elektrische Stimulationskontakt einer anderen Elektrode des Bands entspricht, wobei der eine oder die mehreren elektrischen Stimulationskontakte so konfiguriert sind, dass sie elektrisch mit dem elektrischen Schnittstellenelement verbunden werden können; und
wobei das Gehäuse eine Stimulationstaste (110) umfasst, die so konfiguriert ist, dass sie die Abgabe der Stimulationssignale startet und stoppt;
**dadurch gekennzeichnet, dass**
das Band einen RFID-Tag (224) umfasst, der dafür konfiguriert ist, Informationen bezüglich des Alters oder der Lebensdauer des Bandes zu speichern, um das Aufladen des Gehäuses zu verhindern, wenn das Alter oder die Lebensdauer des Bandes einen Schwellenwert überschreitet.

2. Neurostimulationsvorrichtung (100) nach Anspruch 1, wobei die Stimulationstaste (110) auf der Oberseite (104) des Gehäuses (102) angeordnet ist.

3. Neurostimulationsvorrichtung (100) nach Anspruch 2, wobei die Stimulationstaste (110) durch eine Federeinrichtung (114, 116) nach oben vorgespannt ist und wobei die Federeinrichtung eine Bügel-Federeinrichtung ist, wobei die Bügel-Federeinrichtung eine im Wesentlichen flache Oberfläche und eine Vielzahl von aus der flachen Oberfläche ausgeschnittenen Federfingern (117) umfasst, wobei die Federfinger so konfiguriert sind, dass sie die Stimulationstaste (110) nach oben drücken.

4. Neurostimulationsvorrichtung (100) nach einem der vorhergehenden Ansprüche, wobei das Gehäuse (102) eine oder mehrere zusätzliche Schnittstelleneinrichtungen (110b, 110c) umfasst, die so konfiguriert sind, dass sie es dem Benutzer ermöglichen, einen oder mehrere Stimulationsparameter zu modulieren, wobei die eine oder mehrere zusätzlichen Schnittstelleneinrichtungen auf der Oberseite (104) des Gehäuses angeordnet sind.

5. Neurostimulationsvorrichtung (100) nach einem der vorhergehenden Ansprüche, wobei das Gehäuse (102) einen oder mehrere Ladeanschlüsse (120) umfasst, die so konfiguriert sind, dass sie Strom von einer externen Quelle aufnehmen, um eine im Gehäuse enthaltene wiederaufladbare Batterie (111) aufzuladen, und ferner einen elektrischen Steckverbinder (122) umfasst, der so konfiguriert ist, dass er den einen oder die mehreren Ladeanschlüsse elektrisch mit der wiederaufladbaren Batterie verbindet, wobei derselbe elektrische Steckverbinder die Stimulationstaste (110) elektrisch mit der Schaltung (118) verbindet, die dafür konfiguriert ist, die Stimulationssignale zu erzeugen.

6. Neurostimulationsvorrichtung nach Anspruch 1, wobei das Gehäuse eine Induktionsspule zur drahtlosen Stromversorgung einer im Gehäuse enthaltenen wiederaufladbaren Batterie (111) umfasst.

7. Neurostimulationsvorrichtung (100) nach einem der vorhergehenden Ansprüche, wobei das Gehäuse (102) und das elektrische Schnittstellenelement (122) jeweils Rückleitungselektrodenkontakte umfassen, die so konfiguriert sind, dass sie elektrisch miteinander gekoppelt werden können.

8. Neurostimulationsvorrichtung (100) nach einem der vorhergehenden Ansprüche, wobei das elektrische Schnittstellenelement (122) und das Gehäuse (102) entsprechende Ausrichtungseinrichtungen konfigurieren, die so konfiguriert sind, dass sie die korrekte Ausrichtung des Gehäuses gewährleisten, wenn dieses mit dem Band gekoppelt ist.

9. Neurostimulationsvorrichtung (100) nach einem der vorhergehenden Ansprüche:
wobei das Gehäuse (102) eine Länge, eine Breite und eine Höhe aufweist, wobei die Länge des Gehäuses größer ist als die Breite des Gehäuses;
wobei das Band (150) eine Länge und eine Breite aufweist, wobei die Länge des Bandes größer ist als die Breite des Bandes; und
wobei das Gehäuse (102) dafür konfiguriert ist, mit dem Band (150) gekoppelt zu werden, sodass die Länge des Bandes im Wesentlichen quer zur Breite des Bandes ausgerichtet ist.

10. Neurostimulationsvorrichtung (100) nach einem der vorhergehenden Ansprüche, wobei das Band (150) an einem ersten Ende des Bandes eine Öffnung (156) umfasst, durch die ein zweites Ende des Bandes eingeführt wird, um eine geschlossene Schleife zu bilden, wobei das zweite Ende des Bandes einen Klettverschluss umfasst, um das Band in einer längenverstellbaren Weise an sich selbst zu befestigen, und wobei sich eine Verriegelungsklappe vom Klettverschluss erstreckt, um zu verhindern, dass sich das zweite Ende des Bandes durch die Öffnung zurückzieht und dadurch die geschlossene Schleife öffnet.

11. Neurostimulationsvorrichtung (100) nach einem der vorhergehenden Ansprüche, die ferner eine Seitenwand (108) umfasst, die die erste Oberfläche und die zweite Oberfläche verbindet, wobei die Seitenwand (108) eine erste Seitenwandoberfläche und eine der ersten Seitenwandoberfläche gegenüberliegende zweite Seitenwandoberfläche umfasst, wobei die zweite Seitenwandoberfläche dafür konfiguriert ist, als Stützfläche (112) zu dienen.

12. Tragbares Neurostimulationssystem, wobei das Neurostimulationssystem die Neurostimulationsvorrichtung (100) nach Anspruch 5 oder nach einem der Ansprüche 6-11 umfasst, sofern diese von Anspruch 5 abhängig sind, wobei das System ferner ein Ladegerät (200) umfasst, das so konfiguriert ist, dass es die wiederaufladbare Batterie (111) des Gehäuses elektrisch auflädt, wobei das Ladegerät umfasst:
eine erste Ladegerätfläche (204), eine zweite Ladegerätfläche (206) und eine Seitenwand (208), die sich zwischen der ersten Ladegerätfläche und der zweiten Ladegerätfläche erstreckt;
eine in der ersten Ladegerätfläche gebildete Ladetasche (210), wobei die Tasche so konfiguriert ist, dass sie mindestens einen Teil des Gehäuses (102) aufnimmt, wobei die Ladetasche einen oder mehrere Ladekontakte (212) am Boden der Ladetasche umfasst, die so konfiguriert sind, dass sie eine elektrische Verbindung mit dem Gehäuse herstellen und Energie an dieses übertragen;
und ein Ladekabel, das so konfiguriert ist, dass es an eine externe Stromquelle gekoppelt wird, um über das Ladegerät Strom zu beziehen.

13. Neurostimulationssystem nach Anspruch 12, wobei die Ladetasche (210) eine Öffnung in der ersten Ladegerätfläche (204) umfasst, die eine Oberseite der Ladetasche bildet, und wobei sich die Querschnittsfläche der Ladetasche nach innen verjüngt, während sich die Ladetasche von der Öffnung nach unten erstreckt, wobei die Verjüngung so konfiguriert ist, dass sie das Gehäuse (102) in die Ladetasche einführt.

14. Neurostimulationssystem nach Anspruch 12 oder 13, wobei die Ladetasche (210) so konfiguriert ist, dass sie das Gehäuse (102) aufnimmt, so dass sich die längste Abmessung des Gehäuses von der ersten Ladegerätfläche (104) des Ladegeräts nach oben erstreckt und die kleinste Abmessung des Gehäuses von einer Mitte des Ladegeräts nach außen zeigt.

15. Neurostimulationssystem nach einem der Ansprüche 12 bis 14, wobei die Ladetasche (210) außermittig zum Ladegerät positioniert ist und wobei die Ladetasche so dimensioniert ist, dass das Band auf der ersten Ladegerätfläche (204) des Ladegeräts aufliegt, wenn das Gehäuse (102) geladen wird.

## Revendications

1. Dispositif de neurostimulation à porter sur soi (100) pour la stimulation transcutanée d'un ou plusieurs nerfs périphériques d'un utilisateur, le dispositif comprenant :
un boîtier (102) logeant un circuit (118) configuré pour générer des signaux de stimulation électrique, le boîtier ayant une surface supérieure (104) et une surface inférieure (106), et
un bandeau réglable (150) configuré pour être porté autour du poignet par l'utilisateur, le bandeau ayant un côté intérieur et un côté extérieur, le côté intérieur comprenant au moins une électrode pour chaque nerf à stimuler ;
ledit boîtier (102) étant fixable de manière amovible au bandeau (150),
ladite surface supérieure (104) étant configurée pour être détournée du poignet de l'utilisateur quand le boîtier est fixé au bandeau et que le bandeau est porté autour du poignet de l'utilisateur,
ladite surface supérieure (104) comprenant un écran configuré pour afficher des informations destinée à l'utilisateur,
ladite surface inférieure (106) étant configurée pour être tournée vers le poignet de l'utilisateur quand le boîtier est fixé au bandeau et que le bandeau est porté autour du poignet de l'utilisateur,
ledit bandeau (150) comprenant un élément d'interface électrique (170) et étant configuré pour être couplé électriquement et mécaniquement au boîtier (102),
ledit boîtier (102) comprenant un ou plusieurs contacts de stimulation électrique (124), chaque contact de stimulation électrique correspondant à une électrode différente du bandeau, le ou les contacts de stimulation électrique étant configurés pour être raccordés électriquement à l'élément d'interface électrique, et
ledit boîtier comprenant un bouton de stimulation (110) configuré pour démarrer et arrêter l'émission des signaux de stimulation ;
**caractérisé en ce que**
le bandeau comprend une étiquette RFID (224) configurée pour stocker des informations relatives à l'âge ou à la durée de vie du bandeau de façon à empêcher la charge du boîtier si l'âge ou la durée de vie du bandeau dépasse une limite seuil.

2. Dispositif de neurostimulation (100) selon la revendication 1, dans lequel le bouton de stimulation (110) est positionné sur la surface supérieure (104) du boîtier (102).

3. Dispositif de neurostimulation (100) selon la revendication 2, dans lequel le bouton de stimulation (110) est sollicité vers le haut par un élément ressort (114, 116) et dans lequel l'élément ressort est un élément ressort de support, l'élément ressort de support comprenant une surface globalement plate et une pluralité de languettes ressort (117) découpées dans la surface plate, les languettes ressort étant configurées pour pousser le bouton de stimulation (110) vers le haut.

4. Dispositif de neurostimulation (100) selon l'une quelconque des revendications précédentes, dans lequel le boîtier (102) comprend un ou plusieurs éléments d'interface auxiliaires (110b, 110c) configurés pour permettre à l'utilisateur de moduler un ou plusieurs paramètres de stimulation, le ou les éléments d'interface auxiliaires étant positionnés sur la surface supérieure (104) du boîtier.

5. Dispositif de neurostimulation (100) selon l'une quelconque des revendications précédentes, dans lequel le boîtier (102) comprend un ou plusieurs ports de charge (120) configurés pour recevoir de l'énergie provenant d'une source externe pour charger une batterie rechargeable (111) contenue dans le boîtier, et comprenant en outre un connecteur électrique (122) configuré pour raccorder électriquement le ou les ports de charge à la batterie rechargeable, le même connecteur électrique raccordant électriquement le bouton de stimulation (110) au circuit (118) configuré pour générer les signaux de stimulation.

6. Dispositif de neurostimulation selon la revendication 1, dans lequel le boîtier comprend une bobine d'induction permettant d'alimenter sans fil une batterie rechargeable (111) contenue dans le boîtier.

7. Dispositif de neurostimulation (100) selon l'une quelconque des revendications précédentes, dans lequel le boîtier (102) et l'élément d'interface électrique (122) comprennent chacun des contacts d'électrode de retour configurés pour être couplés électriquement l'un à l'autre.

8. Dispositif de neurostimulation (100) selon l'une quelconque des revendications précédentes, dans lequel l'élément d'interface électrique (122) et le boîtier (102) comprennent des éléments de détrompage correspondants configurés pour assurer la bonne orientation du boîtier quand il est couplé au bandeau.

9. Dispositif de neurostimulation (100) selon l'une quelconque des revendications précédentes :
dans lequel le boîtier (102) présente une longueur, une largeur et une hauteur, la longueur du boîtier étant supérieure à la largeur du boîtier,
dans lequel le bandeau (150) présente une longueur et une largeur, la longueur du bandeau étant supérieure à la largeur du bandeau, et
dans lequel le boîtier (102) est configuré pour être couplé au bandeau (150) de telle façon que la longueur du bandeau soit orientée de manière sensiblement transversale à la longueur du bandeau.

10. Dispositif de neurostimulation (100) selon l'une quelconque des revendications précédentes, dans lequel le bandeau (150) comprend une ouverture (156) sur une première extrémité du bandeau, à travers laquelle une deuxième extrémité du bandeau est insérée pour former une boucle fermée, ladite deuxième extrémité du bandeau comprenant une fermeture autoagrippante pour fixer le bandeau sur lui-même de manière à permettre un réglage de longueur, et un rabat de verrouillage s'étendant à partir de la fermeture autoagrippante pour empêcher la deuxième extrémité du bandeau de reculer à travers l'ouverture pour ouvrir la boucle fermée.

11. Dispositif de neurostimulation (100) selon l'une quelconque des revendications précédentes, comprenant en outre une paroi latérale (108) reliant la première surface et la deuxième surface, ladite paroi latérale (108) comprenant une première surface de paroi latérale et une deuxième surface de paroi latérale opposée à la première surface de paroi latérale, ladite deuxième surface de paroi latérale étant configurée pour être une surface d'appui (112).

12. Système de neurostimulation à porter sur soi, le système de neurostimulation comprenant le dispositif de neurostimulation (100) selon la revendication 5 ou selon l'une quelconque des revendications 6 à 11 dans la mesure où elles dépendent de la revendication 5, le système comprenant en outre un chargeur (200) configuré pour charger électriquement la batterie rechargeable (111) du boîtier, le chargeur comprenant :
une première surface de chargeur (204), une deuxième surface de chargeur (206) et une paroi latérale (208) s'étendant entre la première surface de chargeur et la deuxième surface de chargeur,
un compartiment de charge (210) formé dans la première surface de chargeur, le compartiment étant configuré pour recevoir au moins une partie du boîtier (102), le compartiment de charge comprenant un ou plusieurs contacts de charge (212) au fond du compartiment de charge qui sont configurés pour se coupler électriquement au boîtier et lui transférer de l'énergie,
et un câble de chargement configuré pour être couplé à une source d'alimentation externe pour puiser de l'énergie à travers le chargeur.

13. Système de neurostimulation selon la revendication 12, dans lequel le compartiment de charge (210) comprend une ouverture, dans la première surface de chargeur (204), qui forme une face supérieure du compartiment de charge, et dans lequel la section transversale du compartiment de charge se rétrécit vers l'intérieur à mesure que le compartiment de charge s'étend vers le bas à partir de l'ouverture, le rétrécissement étant configuré pour aider à guider le boîtier (102) dans le compartiment de charge.

14. Système de neurostimulation selon la revendication 12 ou 13, dans lequel le compartiment de charge (210) est configuré pour recevoir le boîtier (102) de telle façon que la plus grande dimension du boîtier s'étende vers le haut à partir de la première surface (104) du chargeur et que la plus petite dimension du boîtier soit orientée vers l'extérieur à partir du centre du chargeur.

15. Système de neurostimulation selon l'une quelconque des revendications 12 à 14, dans lequel le compartiment de charge (210) est positionné à l'écart du centre du chargeur et dans lequel le compartiment de charge est dimensionné de telle façon que le bandeau repose sur la première surface (204) du chargeur quand le boîtier (102) est en cours de charge.
